(19) Europäisches Patentamt
European Patent Office
Office européen des brevets

(11) **EP 4 502 543 A1**

(12) **EUROPEAN PATENT APPLICATION**
published in accordance with Art. 153(4) EPC

(43) Date of publication:
**05.02.2025 Bulletin 2025/06**

(21) Application number: **23778374.1**

(22) Date of filing: **30.03.2023**

(51) International Patent Classification (IPC):
***G01C 22/00*** (2006.01)

(86) International application number:
**PCT/CN2023/085075**

(87) International publication number:
**WO 2023/186009 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **31.03.2022 CN 202210329620**

(71) Applicant: **Vivo Mobile Communication Co., Ltd.
Dongguan, Guangdong 523863 (CN)**

(72) Inventor: **WANG, Feng
Dongguan, Guangdong 523863 (CN)**

(74) Representative: **Conti, Marco
Bugnion S.p.A.
Via di Corticella, 87
40128 Bologna (IT)**

(54) **STEP COUNTING METHOD AND APPARATUS**

(57) This application discloses a step counting method and apparatus. The step counting method includes: obtaining a first acceleration, a second acceleration, and a third acceleration of three axes of an acceleration sensor in a terminal device within a preset time period, where the first acceleration, the second acceleration, and the third acceleration are perpendicular to each other in an acceleration direction; determining a change in motion of a user within the preset time period based on the first acceleration, the second acceleration, and the third acceleration within the preset time period; and in a case that the change in motion of the user within the preset time period is greater than or equal to a preset variance threshold, determining a quantity of steps of the user within the preset time period based on a resultant acceleration of the first acceleration, the second acceleration, and the third acceleration within the preset time period and a gravitational acceleration.

Obtain a first acceleration, a second acceleration, and a third acceleration of three axes of an acceleration sensor in a terminal device within a preset time period, where the first acceleration, the second acceleration, and the third acceleration are perpendicular to each other in an acceleration direction ⟋ S110

Determine a change in motion of a user within the preset time period based on the first acceleration, the second acceleration, and the third acceleration within the preset time period ⟋ S120

In a case that the change in motion of the user within the preset time period is greater than or equal to a preset variance threshold, determine a quantity of steps of the user within the preset time period based on a resultant acceleration of the first acceleration, the second acceleration, and the third acceleration and a gravitational acceleration within the preset time period ⟋ S130

**FIG. 1**

EP 4 502 543 A1

**Description**

**CROSS-REFERENCE TO RELATED APPLICATIONS**

The present invention claims priority to Chinese Patent Application No. 202210329620.4, entitled "STEP COUNTING METHOD AND APPARATUS", and filed with the China National Intellectual Property Administration on March 31, 2022, which is incorporated herein by reference in its entirety.

**TECHNICAL FIELD**

This application belongs to the field of terminal technologies, and specifically, to a step counting method and apparatus.

**BACKGROUND**

An existing step counting algorithm usually collects, by using an acceleration sensor, acceleration data when a user walks, and calculates a quantity of walking steps of the user based on the collected acceleration data. When the user walks or runs on a flat road, since similarity of periodicities of adjacent steps of the user is high, the quantity of walking steps of the user may be calculated with reference to similarity of periodicities of four steps in the acceleration data. In addition, impulses of three axes of an acceleration in the collected acceleration data are stable and do not frequently change directions and turns. In this type of scenario, the quantity of steps calculated by using the existing step counting algorithm is accurate.

But in some special scenarios, such as mountain climbing, rock climbing, walking on an uneven road, or hiking in the wilderness with a weak signal, similarity of periodicities of steps of the user is very low. As a result, accuracy of a quantity of steps calculated by using the existing step counting algorithm is low.

In a process of implementing this application, the inventor discovers that there are at least the following problems in the conventional technology: An existing step counting method has shortcomings such as low accuracy of a calculated quantity of steps and high power consumption in some scenarios such as mountain climbing, rock climbing, walking on an uneven road, or hiking in the wilderness with a weak signal.

**SUMMARY**

Embodiments of this application provide a step counting method and apparatus, to resolve problems such as low accuracy of a calculated quantity of steps and high power consumption in some scenarios such as mountain climbing, rock climbing, walking on an uneven road, or hiking in the wilderness with a weak signal that an existing step counting method has.

To resolve the foregoing technical problems, this application is implemented as follows:

According to a first aspect, an embodiment of this application provides a step counting method, including:

obtaining a first acceleration, a second acceleration, and a third acceleration of three axes of an acceleration sensor in a terminal device within a preset time period, where the first acceleration, the second acceleration, and the third acceleration are perpendicular to each other in an acceleration direction;

determining a change in motion of a user within the preset time period based on the first acceleration, the second acceleration, and the third acceleration within the preset time period; and

in a case that the change in motion of the user within the preset time period is greater than or equal to a preset variance threshold, determining a quantity of steps of the user within the preset time period based on a resultant acceleration of the first acceleration, the second acceleration, and the third acceleration within the preset time period and a gravitational acceleration.

According to a second aspect, an embodiment of this application further provides a step counting apparatus, including:

an obtaining unit, configured to obtain a first acceleration, a second acceleration, and a third acceleration of three axes of an acceleration sensor in a terminal device within a preset time period, where the first acceleration, the second acceleration, and the third acceleration are perpendicular to each other in an acceleration direction;

a first determining unit, configured to determine a change in motion of a user within the preset time period based on the first acceleration, the second acceleration, and the third acceleration within the preset time period; and

a second determining unit, configured to: in a case that the change in motion of the user within the preset time period is greater than or equal to a preset variance threshold, determine a quantity of steps of the user within the preset time period based on a resultant acceleration of the first acceleration, the second acceleration, and the third acceleration within the preset time period and a gravitational acceleration.

According to a third aspect, an embodiment of this application provides an electronic device, where the electronic device includes a processor and a memory, the memory stores a program or an instruction executable on the processor, and the program or the instruction, when executed by the processor, implement the steps of the step counting method according to the first aspect.

According to a fourth aspect, an embodiment of this application provides a readable storage medium, where the readable storage medium stores a program or an instruction, and the program or the instruction, when executed by a processor, implement the steps of the step counting method according to the first aspect.

According to a fifth aspect, an embodiment of this application provides a chip, where the chip includes a processor and a communication interface, the communication interface is coupled to the processor, and the processor is configured to run a program or an instruction, to implement the step counting method according to the first aspect.

According to a sixth aspect, an embodiment of this application provides a computer program product, where the program product is stored in a storage medium, and the program product is executed by at least one processor, to implement the step counting method according to the first aspect.

In embodiments of this application, the change in motion of the user may be determined based on the first acceleration, the second acceleration, and the third acceleration of the three axes of the acceleration sensor in the terminal device carried by the user. In addition, when the change in motion is large, such as when the user hikes in the wilderness with a weak signal or performs some sports whose similarity of periodicities of steps is low, namely, sports with poor movement smoothness, such as mountain climbing, rock climbing, or walking on an uneven road, the actual quantity of steps of the user within the preset time period may be determined directly based on resultant accelerations of accelerations of the three axes in the terminal device carried by the user within the preset time period and the gravitational acceleration. In addition, using the step counting method may further avoid the need to locate and identify the user by using a GPS module when a signal is weak. This reduces unnecessary power consumption.

## BRIEF DESCRIPTION OF THE DRAWINGS

To describe the technical solutions in embodiments of this application more clearly, the following briefly describes the accompanying drawings for describing embodiments of this application. It is clear that the accompanying drawings in the following description show merely some embodiments of this application, and a person of ordinary skill in the art may still derive other drawings from these accompanying drawings without creative efforts.

FIG. 1 is a schematic flowchart of implementation of a step counting method according to an embodiment of this application;

FIG. 2 is a schematic curve diagram of accelerations of three axes of an acceleration sensor and resultant accelerations thereof when a user performs a jumping action in a step counting method according to an embodiment of this application;

FIG. 3 is a schematic curve diagram of accelerations of three axes of an acceleration sensor and resultant accelerations thereof when movement smoothness changes in a step counting method according to an embodiment of this application;

FIG. 4 is a schematic diagram of a structure of a step counting apparatus according to an embodiment of this application;

FIG. 5 is a schematic diagram of a hardware structure of an electronic device according to an embodiment of this application; and

FIG. 6 is a schematic diagram of a structure of an electronic device according to an embodiment of this application.

## DETAILED DESCRIPTION

The technical solutions in embodiments of this application are clearly described below with reference to the accompanying drawings in embodiments of this application. Apparently, the described embodiments are merely some rather than all of embodiments of this application. All other embodiments obtained by a person of ordinary skill in the art based on embodiments of this application shall fall within the protection scope of this application.

The specification and claims of this application, and terms "first" and "second" are used to distinguish similar objects, but are unnecessarily used to describe a specific sequence or order. It may be understood that the data used in such a way is interchangeable in proper circumstances, so that embodiments of this application can be implemented in other sequences than the sequence illustrated or described herein. In addition, objects distinguished by "first" and "second" are usually of a type, and the number of objects is not limited. For example, a first object may be one or more than one. In addition, in the specification and claims, "and/or" means at least one of the connected objects, and the character "/" generally indicates an "or" relationship between associated objects.

A step counting method provided in embodiments of this application is described in detail below by using specific embodiments and application scenarios with reference to the accompanying drawings.

To resolve problems such as low accuracy of a calculated quantity of steps and high power consumption in some scenarios such as mountain climbing, rock climbing, walking on an uneven road, or hiking in the wilderness with a weak signal that an existing step counting method has, this application provides a step counting method. An execution entity of the method may be, but is not limited to, a wearable device such as a smart bracelet or a smart watch, or at least one of user terminals such as a mobile phone and a tablet computer that can be configured to perform the method provided in embodiments of this application.

For ease of description, an implementation of the method is introduced below by using an example in which the execution entity of the method is a terminal device that can perform the method. It may be understood that, the execution entity of the method being a terminal device is merely an example description, and should not be understood as a limitation to the method.

Specifically, the step counting method provided in this application includes: first, obtaining a first acceleration, a second acceleration, and a third acceleration of three axes of an acceleration sensor in a terminal device within a preset time period, where the first acceleration, the second acceleration, and the third acceleration are perpendicular to each other in an acceleration direction; then, determining a change in motion of a user within the preset time period based on the first acceleration, the second acceleration, and the third acceleration within the preset time period; and finally, in a case that the change in motion of the user within the preset time period is greater than or equal to a preset variance threshold, determining a quantity of steps of the user within the preset time period based on a resultant acceleration of the first acceleration, the second acceleration, and the third acceleration within the preset time period and a gravitational acceleration.

In embodiments of this application, the change in motion of the user may be determined based on the first acceleration, the second acceleration, and the third acceleration of the three axes of the acceleration sensor in the terminal device carried by the user. In addition, when the change in motion is large, such as when the user hikes in the wilderness with a weak signal or performs some sports whose similarity of periodicities of steps is low, namely, sports with poor movement smoothness, such as mountain climbing, rock climbing, or walking on an uneven road, the actual quantity of steps of the user within the preset time period may be determined directly based on resultant accelerations of accelerations of the three axes in the terminal device carried by the user within the preset time period and the gravitational acceleration. In addition, using the step counting method may further avoid the need to locate and identify the user by using a global positioning system (Global Positioning System, GPS) module when a signal is weak. This reduces unnecessary power consumption.

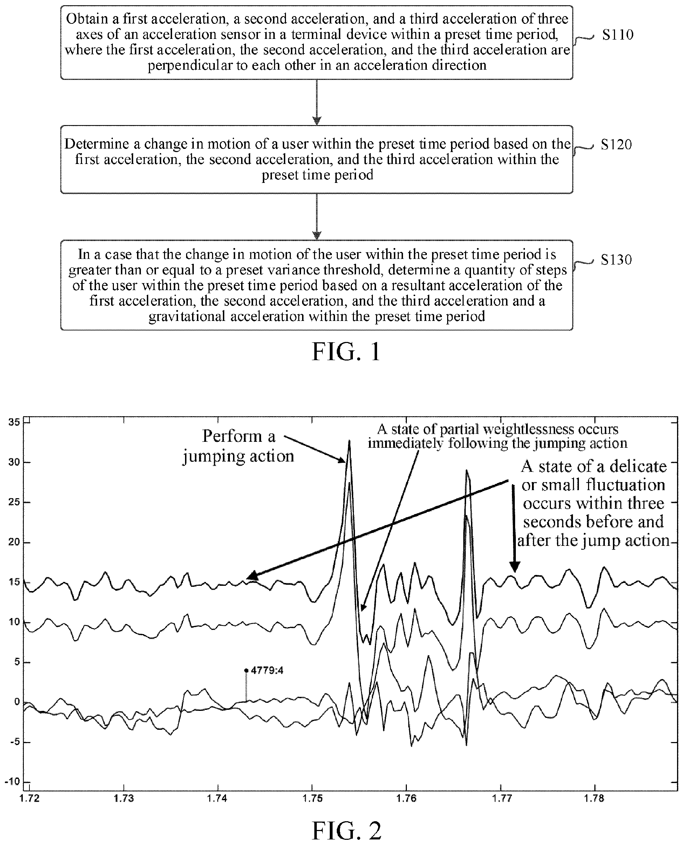

The following describes an implementation process of the method in detail with reference to a schematic flowchart of specific implementation of the step counting method shown in FIG. 1. The following steps are included.

S110: Obtain a first acceleration, a second acceleration, and a third acceleration of three axes of an acceleration sensor in a terminal device within a preset time period, where the first acceleration, the second acceleration, and the third acceleration are perpendicular to each other in an acceleration direction.

Specifically, the first acceleration, the second acceleration, and the third acceleration of the three axes of the acceleration sensor may be accelerations of x, y, and z axes respectively of the acceleration sensor.

It should be understood that, when the user performs some sports such as mountain climbing, rock climbing, or walking on an uneven road whose similarity of periodicities of steps is low, it is often difficult to accurately calculate the actual quantity of steps of the user in an existing manner of calculating the quantity of steps by using the similarity of the periodicities of the adjacent steps. In embodiments of this application, to resolve this problem, the first acceleration, the second acceleration, and the third acceleration of the three axes of the acceleration sensor in the terminal device of the user within a latest preset time period may be obtained, for example, the first acceleration, the second acceleration, and the third acceleration of the three axes of the acceleration sensor within latest 20s may be obtained, to determine whether the user performs a jumping action, and then determine movement smoothness of the user. In this way, it is determined whether to calculate the quantity of steps by using the similarity of the periodicities of the adjacent steps, or to calculate the quantity of steps by using the step counting method proposed in embodiments of this application.

S120: Determine a change in motion of the user within the preset time period based on the first acceleration, the second acceleration, and the third acceleration within the preset time period.

It should be understood that, when the user performs sports such as mountain climbing, rock climbing, or walking on an uneven road whose similarity of periodicities of steps is low, the movement stability of the user is often poor. In embodiments of this application, on this basis, the change in motion of the user within the preset time period is determined based on the first acceleration, the second acceleration, and the third acceleration of the three axes within the preset time period, and then the step counting method is determined, to more accurately calculate the actual quantity of steps of the user.

Optionally, when performing some sports whose similarity of periodicities of steps is low, the user often performs some intermittent jumping actions. In other words, when the user performs the jumping actions, possibility that the movement smoothness of the user is poor is also great. In embodiments of this application, on this basis, whether the user performs some jumping actions within the preset time period may be first determined, and then the movement smoothness of the user within the preset time period is further determined after determining that the user may perform some jumping actions. The determining a change in motion of the user within the preset time period based on the first acceleration, the second acceleration, and the third acceleration within the preset time period includes:

determining a resultant acceleration of a first acceleration, a second acceleration, and a third acceleration at a designated sampling point within the preset time period based on the first acceleration, the second acceleration, and the third acceleration within the preset time period;

determining, based on the resultant acceleration at the designated sampling point within the preset time period, whether the user performs a jumping action within the preset time period; and

in a case that the user performs the jumping action within the preset time period, determining the change in motion of the user within the preset time period based on the first acceleration, the second acceleration, and the third acceleration within the preset time period.

The resultant acceleration of the first acceleration, the second acceleration, and the third acceleration at the designated sampling point within the preset time period is determined based on the first acceleration x, the second acceleration y, and the third acceleration z within the preset time period. Specifically, the resultant acceleration may be obtained by taking a root of a sum of squares of the first acceleration, the second acceleration, and the third acceleration at the designated sampling point within the preset time period. In other words, the resultant acceleration of the first acceleration, the second acceleration, and the third acceleration at each of the designated sampling points within the preset time period is $\sqrt{x^2 + y^2 + z^2}$ . The designated sampling point within the preset time period may be selected based on actual needs. For example, latest N sampling points (namely, N sampling points that are sorted later in time when the sampling points are sorted based on an order of time) within the preset time period may be selected, where N is a positive integer.

Optionally, the determining, based on the resultant acceleration at the designated sampling point within the preset time period, whether the user performs a jumping action within the preset time period includes:

determining movement energy at a designated moment based on a resultant acceleration at a designated quantity of designated sampling points, where the designated quantity of designated sampling points is a sampling point before the designated moment within the preset time period; and

in a case that the movement energy at the designated moment is greater than or equal to a preset energy threshold and a second-order difference of a resultant acceleration at the designated moment is not greater than a preset threshold, determining, based on movement energy at a sampling point within a preset time range before and after the designated moment, whether the user performs the jumping action within the preset time period.

It should be understood that, when the terminal device is in a static state or a smooth movement state, a value of the resultant acceleration of the first acceleration, the second acceleration, and the third acceleration of the three axes of the acceleration sensor in the terminal device should be consistent with a value of a gravitational acceleration. To accurately determine movement smoothness of the terminal device, the gravitational acceleration may be subtracted from the resultant acceleration of the first acceleration, the second acceleration, and the third acceleration of the three axes to obtain a difference between the resultant acceleration and the gravitational acceleration, and then determining is further performed on the difference.

For example, the movement energy at the designated moment may be defined as an average value of a square of a difference between resultant accelerations of first accelerations, second accelerations, and third accelerations of three axes at the first eight sampling points that are adjacent to the designated moment and the gravitational acceleration. Specifically, the first accelerations, the second accelerations, and the third accelerations of the three axes at the first eight sampling points are respectively expressed as (x1, y1, z1), (x2, y2, z2), ..., and (x8, y8, z8). The resultant accelerations of the first accelerations, the second accelerations, and the third accelerations of the three axes at the first eight sampling points that are adjacent to the designated moment are $a1 = \sqrt{x_1^2 + y_1^2 + z_1^2}$, $a2 = \sqrt{x_2^2 + y_2^2 + z_2^2}$, ..., and

$a8 = \sqrt{x_8^2 + y_8^2 + z_8^2}$ . An average value avg of a square of a difference between the resultant accelerations of the first accelerations, the second accelerations, and the third accelerations of the three axes at the first eight sampling points that are adjacent to the designated moment and the gravitational acceleration may be expressed as $avg = ((a1 - g)^2 + (a2 - g)^2 + ... + (a8 - g)^2)/8$.

For example, if the movement energy at the designated moment is greater than or equal to the preset energy threshold and the second-order difference of the resultant acceleration at the designated moment is not greater than the preset threshold, the movement energy at the sampling point within the preset time range before and after the designated moment is determined. Specifically, the preset energy threshold may be set to 20, and the preset threshold may be set to -10. The second-order difference of the resultant acceleration at the designated moment is a first-order difference of a first-order difference of the resultant acceleration at the designated moment. The first-order difference of the resultant acceleration at the designated moment is a previous resultant acceleration at the designated moment minus the resultant acceleration at the designated moment. The second-order difference of the resultant acceleration at the designated moment is a first-order difference of the previous resultant acceleration at the designated moment minus the first-order difference of the resultant acceleration at the designated moment.

For example, determining the movement energy at the sampling point within the preset time range before and after the designated moment may specifically determine movement energy at a sampling point within a preset range before and after the designated moment. For example, if the preset time range is 3s and a sampling frequency is 25 Hz, there are 25 sampling points per second, and the movement energy at the sampling point within the preset time range before and after the designated moment is movement energy at 75 sampling points within 3 seconds before and after the designated moment.

It should be understood that, within the preset time range before and after the user performs a jumping action, the user is often in a static or slightly fluctuating state. Therefore, whether the user is in a state of performing a jumping action at the designated moment may be determined based on a fluctuation amplitude of the movement energy at the sampling point within the preset time range before and after the designated moment. For example, whether the user is in the state of performing the jumping action at the designated moment may be determined based on a quantity of sampling points whose movement energy is less than 0.5 among the 75 sampling points within the 3 seconds before and after the designated moment. Specifically, it may be set that the quantity of sampling points whose movement energy is less than 0.5 among the 75 sampling points within the 3 seconds before and after the designated moment is greater than or equal to 15. In other words, it is determined whether the user is in the state of performing the jumping action at the designated moment. FIG. 2 is a schematic curve diagram of a first acceleration, a second acceleration, and a third acceleration of three axes of an acceleration sensor and resultant accelerations thereof when a user performs a jumping action in a step counting method according to an embodiment of this application. In FIG. 2, it may be learnt that an acceleration value of the user is stable in a period of time before and after the user performs the jumping action, that is, a change in motion is small.

Optionally, the determining a change in motion of the user within the preset time period based on the first acceleration, the second acceleration, and the third acceleration within the preset time period includes:

> determining first accelerations, second accelerations, and third accelerations at a plurality of sampling points from the first acceleration, the second acceleration, and the third acceleration within the preset time period, where the plurality of sampling points are evenly distributed within the preset time period;
> determining resultant accelerations of the first accelerations, the second accelerations, and the third accelerations at the plurality of sampling points based on the first accelerations, the second accelerations, and the third accelerations at the plurality of sampling points;
> determining a variance of a first-order difference of the resultant accelerations at the plurality of sampling points; and
> determining the change in motion of the user within the preset time period based on the variance of the first-order differences of the resultant accelerations at the plurality of sampling points.

For example, the first accelerations, the second accelerations, and the third accelerations of the three axes at the plurality of sampling points are determined from the first acceleration, the second acceleration, and the third acceleration of the three axes within the preset time period. Specifically, the first accelerations, the second accelerations, and the third accelerations of the three axes at the plurality of sampling points (such as 10 sampling points) in a latest period of time (such as within 20s) may be determined from the first acceleration, the second acceleration, and the third acceleration of the three axes within the preset time period. For example, accelerations (Ax1, Ax2, ..., and Ax10, Ay1, Ay2, ..., and Ay10, Az1, Az2, ..., and Az10) of the three axes every 2s in the latest 20s may be determined. Considering that it often takes 2s for a common user to take a complete step, an interval of 2s may be selected as one sampling point.

Then, based on the first accelerations, the second accelerations, and the third accelerations (Ax1, Ax2, ..., and Ax10, Ay1, Ay2, ..., and Ay10, Az1, Az2, ..., and Az10) of the three axes at the foregoing 10 sampling points, the resultant accelerations (Aa1, Aa2, ..., and Aa10) of the first accelerations, the second accelerations, and the third accelerations of the three axes at the plurality of sampling points are determined, where $Aai = \sqrt{Ax_i^2 + Ay_i^2 + Az_i^2}$ , and $i \in [1, 10]$. First-order differences of the resultant accelerations of the first accelerations, the second accelerations, and the third accelerations of the three axes at the 10 sampling points are (Aa2-Aa1, Aa3-Aa2, ..., and Aa10-Aa9), and a variance

$$VAR = \frac{\sum_{i=1}^{10}(Aa_{i+1} - Aa_i - A)^2}{10}$$, where A is an average value of Aa2-Aa1, Aa3-Aa2, ..., and Aa10-Aa9.

The determining the change in motion of the user within the preset time period based on the variance of the first-order differences of the resultant accelerations of the first accelerations, the second accelerations, and the third accelerations of the three axes at the plurality of sampling points includes:

determining that the change in motion of the user within the preset time period is great in a case that the variance of the first-order differences of the resultant accelerations of the first accelerations, the second accelerations, and the third accelerations of the three axes at the plurality of sampling points is greater than a preset variance threshold;
determining a relationship between the variance of the first-order differences of the first accelerations, the second accelerations, and the third accelerations of the three axes at the plurality of sampling points and corresponding preset variance thresholds of the three axes in a case that the variance of the first-order differences of the resultant accelerations of the first accelerations, the second accelerations, and the third accelerations of the three axes at the plurality of sampling points is less than the preset variance threshold; and
determining that the change in motion of the user within the preset time period is great in a case that in the variance of the first-order differences of the first accelerations, the second accelerations, and the third accelerations of the three axes at the plurality of sampling points, there is a variance of first-order differences of accelerations of one or more axes that is greater than the corresponding preset variance threshold.

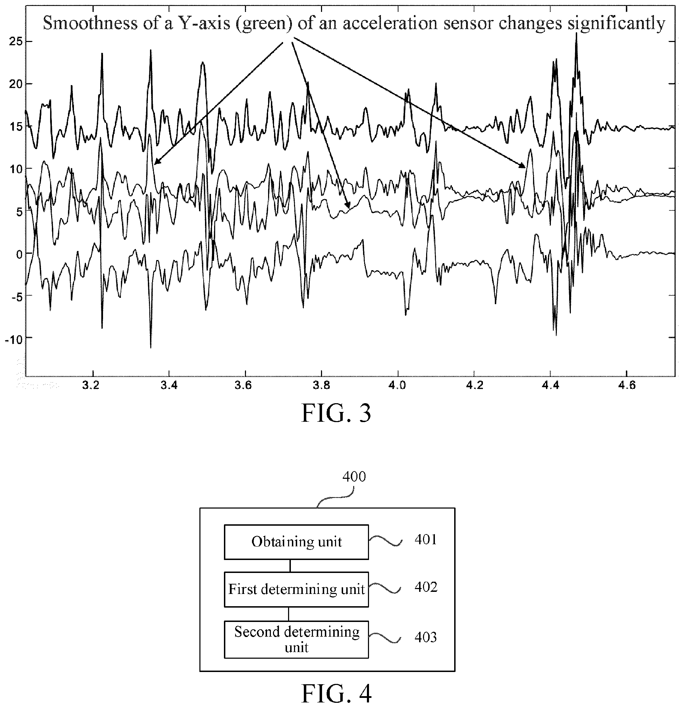

Based on the corresponding preset variance thresholds of the three axes, one variance threshold may be set for each axis. In other words, a preset variance threshold of an x-axis, a preset variance threshold of a y-axis, and a preset variance threshold of a z-axis may be set respectively, or a unified preset variance threshold may be set, which may be set based on an actual situation. FIG. 3 is a schematic curve diagram of accelerations of three axes of an acceleration sensor and resultant accelerations thereof when movement smoothness changes in a step counting method according to an embodiment of this application. It may be learnt from FIG. 3 that, a change in motion of a y-axis of the acceleration sensor is large.

S 130: In a case that a change in motion of a user within a preset time period is greater than or equal to a preset variance threshold, determine a quantity of steps of the user within the preset time period based on a resultant acceleration of a first acceleration, a second acceleration, and a third acceleration within the preset time period and a gravitational acceleration.

Specifically, if the change in motion of the user within the preset time period is greater than or equal to the preset variance threshold, it may be determined that the change in motion of the user within the preset time period is large. In this case, the quantity of steps of the user within the preset time period may be determined based on the resultant acceleration of the first acceleration, the second acceleration, and the third acceleration of the three axes within the preset time period.

Optionally, the determining a quantity of steps of the user within the preset time period based on a resultant acceleration of a first acceleration, a second acceleration, and a third acceleration within the preset time period and a gravitational acceleration includes:

determining resultant accelerations of first accelerations, second accelerations, and third accelerations at a plurality of sampling points within the preset time period; and
determining the quantity of steps of the user within the preset time period based on a cube of a difference between each of the resultant accelerations at the plurality of sampling points within the preset time period and the gravitational acceleration.

For example, an example in which a sampling frequency is 25 Hz is used. A quantity of sampling points per second is 25, and considering that the user often completes a step within two seconds, a quantity of steps of the user within latest two seconds within the preset time period may be determined based on cubes of differences between resultant accelerations at 50 sampling points within the latest two seconds within the preset time period and the gravitational acceleration. It is assumed that the cubes that are of the differences between the resultant accelerations at the 50 sampling points within the latest two seconds within the preset time period and the gravitational acceleration and that are sorted based on an order of time are: 5, 25, 55, 90, 30, -20, -10, 8, 30, 70, -5, and the like.

Optionally, the determining the quantity of steps of the user within the preset time period based on a cube of a difference between each of the resultant accelerations at the plurality of sampling points within the preset time period and the gravitational acceleration includes:

determining a plurality of sets of values that are positive and continuous in time from the cube of the difference between each of the resultant accelerations at the plurality of sampling points within the preset time period and the gravitational acceleration;

determining, from the plurality of sets of values, a quantity of target sets of values among which a sum of each set of values is greater than a set threshold; and

determining the quantity of steps of the user within the preset time period based on the quantity of target sets of values.

After obtaining the cubes that are of the differences between the resultant accelerations at the 50 sampling points within the latest two seconds within the preset time period and the gravitational acceleration and that are sorted based on an order of time being: 5, 25, 55, 90, 30, -20, -10, 8, 30, 70, -5, and the like, a sum of a first set of values that are positive and continuous in time is 5+25+55+90+30=205, which is recorded as step 1, a sum of a second set of values that are positive and continuous in time is 8+30+70=108, which is recorded as step 2, ..., and a sum of a last set of values that are positive and continuous in time is recorded as step N. Assuming that a set threshold is 125, a quantity that is greater than 125 in step 1 to step N may be considered as the quantity of steps of the user within the preset time period.

Optionally, since a limit quantity of steps of a person during walking and running is four steps per second, if the quantity of steps exceeds eight steps within two seconds, an output is eight steps. In other words, if the quantity of steps within the preset time period is greater than a value of the preset time period multiplied by four, the quantity of steps of the user within the preset time period is determined based on the quantity of steps within the preset time period multiplied by four.

Optionally, if the change in motion of the user within the preset time period is greater than or equal to the preset variance threshold, or the user does not perform a jumping action within the preset time period, the actual quantity of steps of the user may be calculated in a normal step counting method. Specifically, a low-pass filter may be used to filter accelerations of the three axes of the acceleration sensor and the resultant accelerations (four axes in total) thereof to obtain an output result; and then, features such as peaks, troughs, height differences, and time differences in accelerations of the four axes are searched after filtering is performed, and features such as a deviation of four adjacent peaks and troughs are calculated.

In a calculation process of the deviation, a maximum value point and a minimum value point are respectively determined as a possible peak and a possible trough from acceleration data of the filtered four axes, then, a height increment dy1 (namely, an amplitude of the peak minus an amplitude of the trough) from the trough to the peak and a height increment dy2 from a next trough to a next peak are calculated, and then, an absolute value of a deviation diff=(dy2-dy1)/(dy2+dy1) is calculated. The greater the value of the deviation diff, the greater the deviation. For example, a preset deviation threshold may be 0.15. If the diff is not greater than the preset deviation threshold, it may be determined that the actual quantity of steps of the user is calculated in the normal step counting method.

According to the step counting method provided in embodiments of this application, the change in motion of the user may be determined based on the first acceleration, the second acceleration, and the third acceleration of the three axes of the acceleration sensor in the terminal device carried by the user. In addition, when the change in motion is large, such as when the user hikes in the wilderness with a weak signal or performs some sports whose similarity of periodicities of steps is low, namely, sports with poor movement smoothness, such as mountain climbing, rock climbing, or walking on an uneven road, the actual quantity of steps of the user within the preset time period may be determined directly based on resultant accelerations of accelerations of the three axes in the terminal device carried by the user within the preset time period and the gravitational acceleration. In addition, using the step counting method may further avoid the need to locate and identify the user by using a GPS module when a signal is weak. This reduces unnecessary power consumption.

It should be noted that, in the step counting method provided in embodiments of this application, an execution entity may be a step counting apparatus. In embodiments of this application, that the step counting apparatus performs the step counting method is used as an example, to describe the step counting apparatus provided in embodiments of this application.

FIG. 4 is a schematic diagram of a structure of a step counting apparatus 400 according to an embodiment of this application. The following components are included.

An obtaining unit 401 is configured to obtain a first acceleration, a second acceleration, and a third acceleration of three axes of an acceleration sensor in a terminal device within a preset time period, where the first acceleration, the second acceleration, and the third acceleration are perpendicular to each other in an acceleration direction;

a first determining unit 402 is configured to determine a change in motion of a user within the preset time period based on the first acceleration, the second acceleration, and the third acceleration within the preset time period; and

a second determining unit 403 is configured to: in a case that the change in motion of the user within the preset time period is greater than or equal to a preset variance threshold, determine a quantity of steps of the user within the preset time period based on a resultant acceleration of the first acceleration, the second acceleration, and the third acceleration within the preset time period and a gravitational acceleration.

Optionally, in an implementation, the second determining unit 403 is configured to:

determine resultant accelerations of first accelerations, second accelerations, and third accelerations at a plurality of sampling points within the preset time period; and
determine the quantity of steps of the user within the preset time period based on a cube of a difference between each of the resultant accelerations at the plurality of sampling points within the preset time period and the gravitational acceleration.

Optionally, in an implementation, the second determining unit 403 is configured to:

determine a plurality of sets of values that are positive and continuous in time from the cube of the difference between each of the resultant accelerations at the plurality of sampling points within the preset time period and the gravitational acceleration;
determine, from the plurality of sets of values, a quantity of target sets of values among which a sum of each set of values is greater than a set threshold; and
determine the quantity of steps of the user within the preset time period based on the quantity of target sets of values.

Optionally, in an implementation, the first determining unit 402 is configured to:

determine a resultant acceleration of a first acceleration, a second acceleration, and a third acceleration at a designated sampling point within the preset time period based on the first acceleration, the second acceleration, and the third acceleration within the preset time period;
determine, based on the resultant acceleration at the designated sampling point within the preset time period, whether the user performs a jumping action within the preset time period; and
in a case that the user performs the jumping action within the preset time period, determine the change in motion of the user within the preset time period based on the first acceleration, the second acceleration, and the third acceleration within the preset time period.

Optionally, in an implementation, the first determining unit 402 is configured to:

determine first accelerations, second accelerations, and third accelerations at a plurality of sampling points from the first acceleration, the second acceleration, and the third acceleration within the preset time period, where the plurality of sampling points are evenly distributed within the preset time period;
determine resultant accelerations of the first accelerations, the second accelerations, and the third accelerations at the plurality of sampling points based on the first accelerations, the second accelerations, and the third accelerations at the plurality of sampling points;
determine a variance of first-order differences of the resultant accelerations at the plurality of sampling points; and
determine the change in motion of the user within the preset time period based on the variance of the first-order differences of the resultant accelerations at the plurality of sampling points.

Optionally, in an implementation, the first determining unit 402 is configured to:

determine movement energy at a designated moment based on a resultant acceleration at a designated quantity of designated sampling points, where the designated quantity of designated sampling points is a sampling point before the designated moment within the preset time period; and
in a case that the movement energy at the designated moment is greater than or equal to a preset energy threshold and a second-order difference of a resultant acceleration at the designated moment is not greater than a preset threshold, determine, based on movement energy at a sampling point within a preset time range before and after the designated moment, whether the user performs the jumping action within the preset time period.

According to the step counting apparatus provided in embodiments of this application, the change in motion of the user may be determined based on the first acceleration, the second acceleration, and the third acceleration of the three axes of the acceleration sensor in the terminal device carried by the user. In addition, when the change in motion is large, such as when the user hikes in the wilderness with a weak signal or performs some sports whose similarity of periodicities of steps is low, namely, sports with poor movement smoothness, such as mountain climbing, rock climbing, or walking on an uneven road, the actual quantity of steps of the user within the preset time period may be determined directly based on resultant accelerations of accelerations of the three axes in the terminal device carried by the user within the preset time period and the gravitational acceleration. In addition, using the step counting method may further avoid the need to locate and identify the user by using a GPS module when a signal is weak. This reduces unnecessary power consumption.

The step counting apparatus in embodiments of this application may be an electronic device, or may be a component in the electronic device, for example, an integrated circuit or a chip. The electronic device may be a terminal, or may be a device other than the terminal. For example, the electronic device may be a mobile phone, a tablet computer, a notebook computer, a handheld computer, a vehicle-mounted electronic device, a mobile internet device (Mobile Internet Device, MID), an augmented reality (augmented reality, AR)/virtual reality (virtual reality, VR) device, a robot, a wearable device, an ultra-mobile personal computer (ultra-mobile personal computer, UMPC), a netbook, a personal digital assistant (personal digital assistant, PDA), or the like, or the electronic device may be a server, a network attached storage (network attached storage, NAS), a personal computer (personal computer, PC), a television (television, TV), a cash machine, a self-service machine, or the like. This is not specifically limited in embodiments of this application.

The step counting apparatus in embodiments of this application may be an apparatus having an operating system. The operating system may be an Android (Android) operating system, may be an ios operating system, or may be another possible operating system. This is not specifically limited in embodiments of this application.

The step counting apparatus provided in embodiments of this application may implement various processes of the method embodiments in FIG. 1 to FIG. 3. This is not described in detail herein again to avoid repetition.

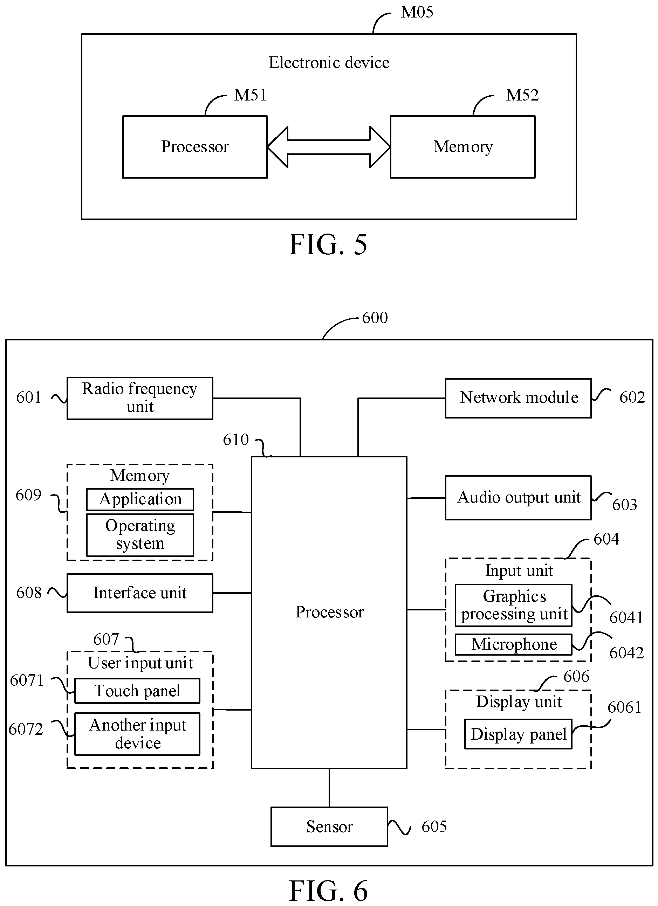

Optionally, as shown in FIG. 5, embodiments of this application further provide an electronic device M05, including a processor M51, a memory M52. The memory M52 stores a program or an instruction executable on the processor M51, and the program or the instruction, when executed by the processor M51, implement the various processes of the step counting method embodiment, and may implement the same technical effect. This is not described in detail herein again to avoid repetition.

It should be noted that, the electronic device in embodiments of this application includes the mobile electronic device and the non-mobile electronic device.

FIG. 6 is a schematic diagram of a hardware structure of an electronic device according to an embodiment of this application.

The electronic device 600 includes, but is not limited to, components such as a radio frequency unit 601, a network module 602, an audio output unit 603, an input unit 604, a sensor 605, a display unit 606, a user input unit 607, an interface unit 608, a memory 609, and a processor 610.

A person skilled in the art may understand that the electronic device 600 may further include the power supply (such as a battery) for supplying power to the components. The power supply may be logically connected to the processor 610 by a power management system, thereby implementing functions such as charging, discharging, and power consumption management by using the power management system. An electronic structure shown in FIG. 6 does not constitute a limitation to the electronic device, and the electronic device may include more or fewer components than those shown in the figure, or some components may be combined, or a different component deployment may be used, and details are not repeated.

The processor 610 obtains a first acceleration, a second acceleration, and a third acceleration of three axes of an acceleration sensor in a terminal device within a preset time period, where the first acceleration, the second acceleration, and the third acceleration are perpendicular to each other in an acceleration direction; determines a change in motion of a user within the preset time period based on the first acceleration, the second acceleration, and the third acceleration within the preset time period; and in a case that the change in motion of the user within the preset time period is greater than or equal to a preset variance threshold, determines a quantity of steps of the user within the preset time period based on a resultant acceleration of the first acceleration, the second acceleration, and the third acceleration within the preset time period and a gravitational acceleration.

Optionally, the processor 610 is further configured to: determine resultant accelerations of first accelerations, second accelerations, and third accelerations at a plurality of sampling points within the preset time period; and determine the quantity of steps of the user within the preset time period based on a cube of a difference between each of the resultant accelerations at the plurality of sampling points within the preset time period and the gravitational acceleration.

Optionally, the processor 610 is further configured to: determine a plurality of sets of values that are positive and continuous in time from the cube of the difference between each of resultant accelerations at the plurality of sampling points within the preset time period and the gravitational acceleration; determine, from the plurality of sets of values, a quantity of target sets of values among which a sum of each set of values is greater than a set threshold; and determine the quantity of steps of the user within the preset time period based on the quantity of target sets of values.

Optionally, the processor 610 is further configured to: determine a resultant acceleration of a first acceleration, a second acceleration, and a third acceleration at a designated sampling point within the preset time period based on the first acceleration, the second acceleration, and the third acceleration within the preset time period; determine, based on the resultant acceleration at the designated sampling point within the preset time period, whether the user performs a jumping action within the preset time period; and in a case that the user performs the jumping action within the preset time period, determine the change in motion of the user within the preset time period based on the first acceleration, the second acceleration, and the third acceleration within the preset time period.

Optionally, the processor 610 is further configured to: determine first accelerations, second accelerations, and third accelerations at a plurality of sampling points from the first acceleration, the second acceleration, and the third acceleration within the preset time period, where the plurality of sampling points are evenly distributed within the preset time period; determine resultant accelerations of the first accelerations, the second accelerations, and the third accelerations at the plurality of sampling points based on the first accelerations, the second accelerations, and the third accelerations at the plurality of sampling points; determine a variance of first-order differences of the resultant accelerations at the plurality of sampling points; and determine the change in motion of the user within the preset time period based on the variance of the first-order differences of the resultant accelerations at the plurality of sampling points.

Optionally, the processor 610 is further configured to: determine movement energy at a designated moment based on a resultant acceleration at a designated quantity of designated sampling points, where the designated quantity of designated sampling points is a sampling point before the designated moment within the preset time period; and in a case that the movement energy at the designated moment is greater than or equal to a preset energy threshold and a second-order difference of a resultant acceleration at the designated moment is not greater than a preset threshold, determine, based on movement energy at a sampling point within a preset time range before and after the designated moment, whether the user performs the jumping action within the preset time period.

An electronic device provided in embodiments of this application is used. The change in motion of the user may be determined based on the first acceleration, the second acceleration, and the third acceleration of the three axes of the acceleration sensor in the terminal device carried by the user. In addition, when the change in motion is large, such as when the user hikes in the wilderness with a weak signal or performs some sports whose similarity of periodicities of steps is low, namely, sports with poor movement smoothness, such as mountain climbing, rock climbing, or walking on an uneven road, the actual quantity of steps of the user within the preset time period may be determined directly based on resultant accelerations of accelerations of the three axes in the terminal device carried by the user within the preset time period and the gravitational acceleration. In addition, using the step counting method may further avoid the need to locate and identify the user by using a GPS module when a signal is weak. This reduces unnecessary power consumption.

It should be understood that, in embodiments of this application, the input unit 604 may include a graphics processing unit (Graphics Processing Unit, GPU) 6041 and a microphone 6042. The graphics processing unit 6041 performs processing on image data of a static picture or a video that is obtained by an image acquisition apparatus (for example, a camera) in a video acquisition mode or an image acquisition mode. The display unit 606 may include a display panel 6061, and the display panel 6061 may be configured by using a liquid crystal display, an organic light-emitting diode, or the like. The user input unit 607 includes at least one of a touch panel 6071 and another input device 6072. The touch panel 6071 is also referred to as a touchscreen. The touch panel 6071 may include two parts: a touch detection apparatus and a touch processor. The another input device 6072 may include, but not limited to, a physical keyboard, a functional key (such as a volume control key or a switch key), a track ball, a mouse, and a joystick, which are not described herein in detail.

The memory 609 may be configured to store a software program and various data. The memory 609 may mainly include a first storage area storing a program or an instruction and a second storage area storing data. The first storage area may store an operating system, an application program or an instruction required by at least one function (such as a sound playback function and an image display function), and the like. In addition, the memory 609 may include a volatile memory or a non-volatile memory, or the memory 609 may include both a volatile memory and a non-volatile memory. The non-volatile memory may be a read-only memory (Read-Only Memory, ROM), a programmable read-only memory (Programmable ROM, PROM), an erasable programmable read-only memory (Erasable PROM, EPROM), an electrically erasable programmable read-only memory (Electrically EPROM, EEPROM), or a flash memory. The volatile memory may be a random access memory (Random Access Memory, RAM), a static random access memory (Static RAM, SRAM), a dynamic random access memory (Dynamic RAM, DRAM), a synchronous dynamic random access memory (Synchronous DRAM, SDRAM), a double data rate synchronous dynamic random access memory (Double Data Rate SDRAM, DDRSDRAM), an enhanced synchronous dynamic random access memory (Enhanced SDRAM, ESDRAM), a synch link dynamic random access memory (Synch link DRAM, SLDRAM), and a direct rambus random access memory (Direct Rambus RAM, DR RAM). The memory 609 in embodiments of this application includes but is not limited to these memories and any other suitable types of memories.

The processor 610 may include one or more processing units; and optionally, the processor 610 integrates an application processor and a modem processor. The application processor mainly processes operations relating to an operating system, a user interface, an application program, and the like. The modem processor mainly processes a wireless communication signal, such as a baseband processor. It may be understood that, the foregoing modem processor may also not be integrated into the processor 610.

Embodiments of this application further provide a readable storage medium, where the readable storage medium stores a program or an instruction. The program or the instruction, when executed by a processor, implements various processes of the step counting method embodiment, and may implement the same technical effect. This is not described in detail herein again to avoid repetition.

The processor is a processor in the electronic device described in the foregoing embodiment. The readable storage medium includes a computer-readable storage medium, for example, a read-only memory (Read-Only Memory, ROM), a random access memory (Random Access Memory, RAM), a magnetic disk, or an optical disc.

Embodiments of this application further provide a chip. The chip includes a processor and a communication interface. The communication interface is coupled to the processor. The processor is configured to run a program or an instruction, to implement various processes of the step counting method embodiment, and may implement the same technical effect. This is not described in detail herein again to avoid repetition.

It should be understood that, the chip mentioned in embodiments of this application may be further referred to as a system-level chip, a system chip, a chip system, a system-on-chip, or the like.

Embodiments of this application provide a computer program product, where the program product is stored in a storage medium, and the program product is executed by at least one processor, to implement various processes of the step counting method embodiment, and may implement the same technical effect. This is not described in detail herein again to avoid repetition.

It should be noted that, the terms "include", "comprise" or any other variants mean to cover the non-exclusive inclusion, so that the processes, methods, objects, or apparatuses which include a series of elements not only include those elements, but also include other elements which are not clearly listed, or include inherent elements of the processes, methods, objects, or apparatuses. Without more limitations, elements defined by the sentence "including one" does not exclude that there are still other same elements in the processes, methods, objects, or apparatuses. In addition, it should be noted that, the scope of the methods and apparatuses in the implementations of this application is not limited to performing functions in the order shown or discussed, and may further include performing functions in a substantially simultaneous manner or in a reverse order according to the functions involved, for example, the described methods may be performed in an order different from the order described, and various steps may be added, omitted, or combined. In addition, features described with reference to some examples may be combined in other examples.

Through the foregoing description on the implementations, a person skilled in the art can clearly learn that the foregoing embodiment methods may be implemented by using software in combination with a necessary universal hardware platform. Certainly, the embodiment methods may also be implemented by using hardware, but the former is a better implementation in many cases. Based on such an understanding, the technical solutions of this application essentially, or the part contributing to the related art, may be presented in the form of a computer software product. The computer software product is stored in a storage medium (for example, a ROM/RAM, a magnetic disk, or an optical disc) including several instructions to enable a terminal (which may be a mobile phone, a computer, a server, an air conditioner, a network device, or the like) to perform the methods described in embodiments of this application.

Although embodiments of this application have been described above with reference to the accompanying drawings, this application is not limited to the specific implementations described above, and the specific implementations described above are merely examples and not limitative. A person of ordinary skill in the art may make various variations under the teaching of this application without departing from the spirit of this application and the protection scope of the claims, and such variations shall all fall within the protection scope of this application.

## Claims

1. A step counting method, comprising:

   obtaining a first acceleration, a second acceleration, and a third acceleration of three axes of an acceleration sensor in a terminal device within a preset time period, wherein the first acceleration, the second acceleration, and the third acceleration are perpendicular to each other in an acceleration direction;
   determining a change in motion of a user within the preset time period based on the first acceleration, the second acceleration, and the third acceleration within the preset time period; and
   in a case that the change in motion of the user within the preset time period is greater than or equal to a preset variance threshold, determining a quantity of steps of the user within the preset time period based on a resultant acceleration of the first acceleration, the second acceleration, and the third acceleration within the preset time period and a gravitational acceleration.

2. The method according to claim 1, wherein the determining a quantity of steps of the user within the preset time period based on a resultant acceleration of the first acceleration, the second acceleration, and the third acceleration within the preset time period and a gravitational acceleration comprises:

determining resultant accelerations of first accelerations, second accelerations, and third accelerations at a plurality of sampling points within the preset time period; and

determining the quantity of steps of the user within the preset time period based on a cube of a difference between each of the resultant accelerations at the plurality of sampling points within the preset time period and the gravitational acceleration.

3. The method according to claim 2, wherein the determining the quantity of steps of the user within the preset time period based on a cube of a difference between each of the resultant accelerations at the plurality of sampling points within the preset time period and the gravitational acceleration comprises:

determining a plurality of sets of values that are positive and continuous in time from the cube of the difference between each of the resultant accelerations at the plurality of sampling points within the preset time period and the gravitational acceleration;

determining, from the plurality of sets of values, a quantity of target sets of values among which a sum of each set of values is greater than a set threshold; and

determining the quantity of steps of the user within the preset time period based on the quantity of target sets of values.

4. The method according to claim 1, wherein the determining a change in motion of a user within the preset time period based on the first acceleration, the second acceleration, and the third acceleration within the preset time period comprises:

determining a resultant acceleration of a first acceleration, a second acceleration, and a third acceleration at a designated sampling point within the preset time period based on the first acceleration, the second acceleration, and the third acceleration within the preset time period;

determining, based on the resultant acceleration at the designated sampling point within the preset time period, whether the user performs a jumping action within the preset time period; and

in a case that the user performs the jumping action within the preset time period, determining the change in motion of the user within the preset time period based on the first acceleration, the second acceleration, and the third acceleration within the preset time period.

5. The method according to claim 1 or 4, wherein the determining a change in motion of a user within the preset time period based on the first acceleration, the second acceleration, and the third acceleration within the preset time period comprises:

determining first accelerations, second accelerations, and third accelerations at a plurality of sampling points from the first acceleration, the second acceleration, and the third acceleration within the preset time period, wherein the plurality of sampling points are evenly distributed within the preset time period;

determining resultant accelerations of the first accelerations, the second accelerations, and the third accelerations at the plurality of sampling points based on the first accelerations, the second accelerations, and the third accelerations at the plurality of sampling points;

determining a variance of first-order differences of the resultant accelerations at the plurality of sampling points; and

determining the change in motion of the user within the preset time period based on the variance of the first-order differences of the resultant accelerations at the plurality of sampling points.

6. The method according to claim 4, wherein the determining, based on the resultant acceleration at the designated sampling point within the preset time period, whether the user performs a jumping action within the preset time period comprises:

determining movement energy at a designated moment based on a resultant acceleration at a designated quantity of designated sampling points, wherein the designated quantity of designated sampling points is a sampling point before the designated moment within the preset time period; and

in a case that the movement energy at the designated moment is greater than or equal to a preset energy threshold and a second-order difference of a resultant acceleration at the designated moment is not greater than a preset threshold, determining, based on movement energy at a sampling point within a preset time range before and after the designated moment, whether the user performs the jumping action within the preset time period.

7. A step counting apparatus, comprising:

an obtaining unit, configured to obtain a first acceleration, a second acceleration, and a third acceleration of three axes of an acceleration sensor in a terminal device within a preset time period, wherein the first acceleration, the second acceleration, and the third acceleration are perpendicular to each other in an acceleration direction;
a first determining unit, configured to determine a change in motion of a user within the preset time period based on the first acceleration, the second acceleration, and the third acceleration within the preset time period; and
a second determining unit, configured to: in a case that the change in motion of the user within the preset time period is greater than or equal to a preset variance threshold, determine a quantity of steps of the user within the preset time period based on a resultant acceleration of the first acceleration, the second acceleration, and the third acceleration within the preset time period and a gravitational acceleration.

8. The apparatus according to claim 7, wherein the second determining unit is configured to:

determine resultant accelerations of first accelerations, second accelerations, and third accelerations at a plurality of sampling points within the preset time period; and
determine the quantity of steps of the user within the preset time period based on a cube of a difference between each of the resultant accelerations at the plurality of sampling points within the preset time period and the gravitational acceleration.

9. The apparatus according to claim 8, wherein the second determining unit is configured to:

determine a plurality of sets of values that are positive and continuous in time from the cube of the difference between each of the resultant accelerations at the plurality of sampling points within the preset time period and the gravitational acceleration;
determine, from the plurality of sets of values, a quantity of target sets of values among which a sum of each set of values is greater than a set threshold; and
determine the quantity of steps of the user within the preset time period based on the quantity of target sets of values.

10. The apparatus according to claim 7, wherein the first determining unit is configured to:

determine a resultant acceleration of a first acceleration, a second acceleration, and a third acceleration at a designated sampling point within the preset time period based on the first acceleration, the second acceleration, and the third acceleration within the preset time period;
determine, based on the resultant acceleration at the designated sampling point within the preset time period, whether the user performs a jumping action within the preset time period; and
in a case that the user performs the jumping action within the preset time period, determine the change in motion of the user within the preset time period based on the first acceleration, the second acceleration, and the third acceleration within the preset time period.

11. The apparatus according to claim 7 or 10, wherein the first determining unit is configured to:

determine first accelerations, second accelerations, and third accelerations at a plurality of sampling points from the first acceleration, the second acceleration, and the third acceleration within the preset time period, wherein the plurality of sampling points are evenly distributed within the preset time period;
determine resultant accelerations of the first accelerations, the second accelerations, and the third accelerations at the plurality of sampling points based on the first accelerations, the second accelerations, and the third accelerations at the plurality of sampling points;
determine a variance of first-order differences of the resultant accelerations at the plurality of sampling points; and
determine the change in motion of the user within the preset time period based on the variance of the first-order differences of the resultant accelerations at the plurality of sampling points.

12. The apparatus according to claim 10, wherein the first determining unit is configured to:

determine movement energy at a designated moment based on a resultant acceleration at a designated quantity of designated sampling points, wherein the designated quantity of designated sampling points is a sampling point before the designated moment within the preset time period; and

in a case that the movement energy at the designated moment is greater than or equal to a preset energy threshold and a second-order difference of a resultant acceleration at the designated moment is not greater than a preset threshold, determine, based on movement energy at a sampling point within a preset time range before and after the designated moment, whether the user performs the jumping action within the preset time period.

13. An electronic device, comprising a processor and a memory, wherein the memory stores a program or an instruction executable on the processor, and the program or the instruction, when executed by the processor, implement the steps of the step counting method according to any one of claims 1 to 6.

14. A readable storage medium, wherein the readable storage medium stores a program or an instruction, and the program or the instruction, when executed by a processor, implement the steps of the step counting method according to any one of claims 1 to 6.

15. A chip, wherein the chip comprises a processor and a communication interface, the communication interface is coupled to the processor, and the processor is configured to run a program or an instruction, to implement the steps of the step counting method according to any one of claims 1 to 6.

16. A computer program product, wherein the program product is executed by at least one processor to implement the steps of the step counting method according to any one of claims 1 to 6.

17. An electronic device, wherein the electronic device is configured to perform the steps of the step counting method according to any one of claims 1 to 6.

Obtain a first acceleration, a second acceleration, and a third acceleration of three axes of an acceleration sensor in a terminal device within a preset time period, where the first acceleration, the second acceleration, and the third acceleration are perpendicular to each other in an acceleration direction — S110

Determine a change in motion of a user within the preset time period based on the first acceleration, the second acceleration, and the third acceleration within the preset time period — S120

In a case that the change in motion of the user within the preset time period is greater than or equal to a preset variance threshold, determine a quantity of steps of the user within the preset time period based on a resultant acceleration of the first acceleration, the second acceleration, and the third acceleration and a gravitational acceleration within the preset time period — S130

FIG. 1

FIG. 2

Smoothness of a Y-axis (green) of an acceleration sensor changes significantly

FIG. 3

FIG. 4

M05

Electronic device

M51

Processor

M52

Memory

FIG. 5

600

601 Radio frequency unit

602 Network module

610

609 Memory
Application
Operating system

603 Audio output unit

604 Input unit
Graphics processing unit 6041
Microphone 6042

608 Interface unit

Processor

607 User input unit
6071 Touch panel
6072 Another input device

606 Display unit
Display panel 6061

605 Sensor

FIG. 6

<table>
<tr><td colspan="2" align="center"><b>INTERNATIONAL SEARCH REPORT</b></td><td colspan="2">International application No.<br><br><b>PCT/CN2023/085075</b></td></tr>
</table>

**A. CLASSIFICATION OF SUBJECT MATTER**

G01C22/00 (2006.01)i

According to International Patent Classification (IPC) or to both national classification and IPC

**B. FIELDS SEARCHED**

Minimum documentation searched (classification system followed by classification symbols)

IPC: G01C 22

Documentation searched other than minimum documentation to the extent that such documents are included in the fields searched

Electronic data base consulted during the international search (name of data base and, where practicable, search terms used)

CNTXT, VCN, ENTXTC, VEN: 步数, 计步, 加速度, 加速率, 重力, 垂直, 竖直, 运动量, 跳跃, 爬山, 攀岩, 浮动, 变化, 起伏, 波动, 周期, 紊乱, 不规律, 不规则, step?, pedometer?, count+, accelerat+, gravity

**C. DOCUMENTS CONSIDERED TO BE RELEVANT**

| Category* | Citation of document, with indication, where appropriate, of the relevant passages | Relevant to claim No. |
|---|---|---|
| PX | CN 114719883 A (VIVO COMMUNICATION TECHNOLOGY CO., LTD.) 08 July 2022 (2022-07-08)<br>  description paragraphs [0007]-[0127], claims 1-14, and figures 1-6 | 1-17 |
| A | CN 106225803 A (INSPUR SOFTWARE GROUP CO., LTD.) 14 December 2016 (2016-12-14)<br>  description, paragraphs [0015]-[0032] | 1-17 |
| A | CN 102445214 A (HANGZHOU DIANZI UNIVERSITY) 09 May 2012 (2012-05-09)<br>  entire document | 1-17 |
| A | CN 104197952 A (BAIDU ONLINE NETWORK TECHNOLOGY (BEIJING) CO., LTD.) 10 December 2014 (2014-12-10)<br>  entire document | 1-17 |
| A | CN 114034313 A (GUANGDONG TRANSTEK MEDICAL ELECTRONICS CO., LTD.) 11 February 2022 (2022-02-11)<br>  entire document | 1-17 |
| A | US 2010024531 A1 (KYOCERA CORP.) 04 February 2010 (2010-02-04)<br>  entire document | 1-17 |

☑ Further documents are listed in the continuation of Box C.       ☑ See patent family annex.

| | |
|---|---|
| \* Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |
| Date of the actual completion of the international search<br><br>**17 July 2023** | Date of mailing of the international search report<br><br>**20 July 2023** |
| Name and mailing address of the ISA/CN<br><br>**China National Intellectual Property Administration (ISA/CN)**<br>**China No. 6, Xitucheng Road, Jimenqiao, Haidian District, Beijing 100088** | Authorized officer<br><br><br><br>Telephone No. |

Form PCT/ISA/210 (second sheet) (July 2022)

**INTERNATIONAL SEARCH REPORT**

| International application No. |
|---|
| **PCT/CN2023/085075** |

**C.    DOCUMENTS CONSIDERED TO BE RELEVANT**

| Category* | Citation of document, with indication, where appropriate, of the relevant passages | Relevant to claim No. |
|---|---|---|
| A | JP 2011014157 A (KYOCERA CORP.) 20 January 2011 (2011-01-20)<br>        entire document | 1-17 |

Form PCT/ISA/210 (second sheet) (July 2022)

**INTERNATIONAL SEARCH REPORT**
Information on patent family members

International application No.

**PCT/CN2023/085075**

| Patent document cited in search report | | | Publication date (day/month/year) | Patent family member(s) | | | Publication date (day/month/year) |
|---|---|---|---|---|---|---|---|
| CN | 114719883 | A | 08 July 2022 | None | | | |
| CN | 106225803 | A | 14 December 2016 | None | | | |
| CN | 102445214 | A | 09 May 2012 | None | | | |
| CN | 104197952 | A | 10 December 2014 | None | | | |
| CN | 114034313 | A | 11 February 2022 | None | | | |
| US | 2010024531 | A1 | 04 February 2010 | US | 2012295552 | A1 | 22 November 2012 |
| | | | | US | 8561457 | B2 | 22 October 2013 |
| | | | | JP | 2010033337 | A | 12 February 2010 |
| | | | | JP | 4644274 | B2 | 02 March 2011 |
| | | | | US | 8276434 | B2 | 02 October 2012 |
| JP | 2011014157 | A | 20 January 2011 | JP | 5216825 | B2 | 19 June 2013 |

Form PCT/ISA/210 (patent family annex) (July 2022)

**REFERENCES CITED IN THE DESCRIPTION**

**Patent documents cited in the description**

- CN 202210329620